# FASCICULE DE BREVET EUROPEEN

(11) **EP 0 842 282 B1**
(45) Date de publication et mention de la délivrance du brevet: **07.09.2005**
(21) Numéro de dépôt: 96926424.1
(22) Date de dépôt: 18.07.1996
(51) Int. Cl.: C12N 15/62, C07K 14/47, A61K 38/17, C12N 5/10, C12N 1/21

(54) **PROTEINES HETERO-MULTIMERIQUES RECOMBINANTES DU TYPE ALPHA-BETA C4BP**
REKOMBINANTE HETERO-MULTIMERISCHE PROTEINE VON DEM ALPHA-BETA C4 BP TYP
ALFA-BETA C4BP-TYPE RECOMBINANT HETEROMULTIMERIC PROTEINS

(30) Priorité: 21.07.1995 FR 9508901
(43) Date de publication de la demande: 20.05.1998
(73) Titulaire: UNIVERSITE PIERRE ET MARIE CURIE, 75252 Paris Cédex 05 (FR); Université de Reims Champagne-Ardennes, 51097 Reims (FR)
(72) Inventeur: KLATZMANN, David, F-75013 Paris (FR); COHEN, Jacques, F-51100 Reims (FR)
(74) Mandataire: Desaix, Anne
(86) Numéro de dépôt international: PCT/FR1996/001132
(87) Numéro de publication internationale: WO 1997/004109

(56) Documents cités:
- WO-A-91/11461
- J BIOL CHEM, FEB 15 1993, 268 (5) P3033-6, UNITED STATES, XP002013808 ANDERSSON A ET AL: "High affinity binding of human vitamin K-dependent protein S to a truncated recombinant beta-chain of C4b-binding protein expressed in Escherichia coli."

## Description

La présente invention porte sur des protéines de fusion hétéro-multimériques de type C4BP, les compositions les contenant ainsi que leur procédé de préparation. Plus particulièrement, cette invention est relative à des protéines de fusion hétéro-multimériques issues de l'association de monomères α et β de la protéine C4BP ou de fragments de ceux-ci, ces monomères étant fusionnés à des polypeptides issus de protéines à activité fonctionnelle ayant des propriétés de ligand ou de récepteur.

Les numéros entre parenthèse sont relatifs à la liste bibliographique en fin de texte.

La « C4BP- binding protein » (C4BP) appelée auparavant protéine riche en proline, est une protéine importante à la fois dans le système de la coagulation (1), et dans le système du complément (2, 3). La forme majoritaire de la C4BP est composée de 7 chaînes α identiques de 75 Kd et d'une chaîne β de 45 Kd. La séquence de nucléotide du cDNA et la séquence protéique de la chaîne α ont été déterminées (4). Une description complète de la C4BP humaine, son isolement et sa caractérisation ont été décrits dans (2) ; une mise à jour des connaissances sur cette molécule a été synthétisée dans (5).

L'existence des deux chaînes α et β était inconnue jusqu'en 1990, ce n'est qu'en 1990 que A. Hillarp a mis en évidence pour la première fois l'existence dans la protéine multimérique une nouvelle sous-unité désignée chaîne β, et qui contient le site de liaison à la protéine S (6, 7).

La demande de brevet BIOGEN WO 91/11461 décrit des protéines multimériques du type C4BP uniquement constituées de monomères α dans lesquels la partie N-terminale a été substituée par un fragment de la protéine CD4.

Cependant, les constructions décrites dans ce document ne mettent en oeuvre que la chaîne α, la chaîne β étant inconnue à cette époque.

L'inconvénient de ces constructions est qu'il est extrêmement difficile de contrôler l'état physique de la molécule synthétique c'est-à-dire le nombre de monomères qui s'associent d'une part, et d'autre part lorsque l'on souhaite associer au sein du multimère deux parties fonctionnelles différentes, de contrôler les proportions de ces éléments fonctionnels.

Des agents thérapeutiques ayant une fonction au niveau du système immunitaire, qu'il soit cellulaire ou humoral, ou agent d'immuno-intervention, se sont développés dans de nombreuses directions ; les champs d'application des immuno-interventions thérapeutiques des anticorps surtout des anticorps monoclonaux restent cependant aujourd'hui modestes, du fait de la mauvaise maîtrise des phénomènes survenants après leur liaison cellulaire. En effet, même humanisés, on ne sait aujourd'hui utiliser ces anticorps qu'à des fins de destruction cellulaire.

Le développement d'anticorps bi-spécifiques a également été envisagé et certains d'entre eux comportent une partie susceptible de se lier à un antigène et l'autre partie ayant une qualité de ligand vis-à-vis d'un récepteur permettant d'effectuer le routage vers un système cellulaire (8). Cependant, ces systèmes ne permettent pas d'associer plusieurs ligands dans un même complexe, ce qui dans certains cas semble nécessaire au déclenchement d'une réponse immunitaire.

Un autre système multimérisant proposé repose sur le Fc de l'IgM ; il possède plusieurs inconvénients : le principal est de réaliser des espèces moléculaires de tailles variables laissant des résidus sulfhydriles libres capables de réagir avec des molécules plasmatiques ou des surfaces cellulaires. En outre, les fonctions du fragment Fc de liaison au récepteur cellulaire et d'activation du complément peuvent être indésirables.

Un molécule hétéro-multimérique recombinante peut permettre au contraire d'associer plusieurs fonctions anticorps, ou plusieurs molécules d'enzymes ou plusieurs antigènes ou des fragments ou des mélanges de ceux-ci, réalisant un traceur multi-valent ayant un meilleur potentiel d'amplification du signal détecté en comparaison d'une protéine de fusion associant un seul anticorps ou un anticorps bi-spécifique et une molécule enzymatique ou antigénique.

Cette approche peut permettre d'envisager, outre l'opsonisation par déclenchement de l'immunité cellulaire, la mise en oeuvre provoquée de l'immunité humorale par l'activation du complément.

L'objectif de la présente invention est donc de développer des molécules chimériques recombinantes solubles hétéro-multimériques associant des fonctions différentes dans une même molécule, dans une perspective d'immuno-intervention en pathologie dysimmunitaire humaine. Ces molécules permettront d'intervenir dans les mécanismes physiopathologiques des différentes affections, notamment dans les domaines relatifs :
- à la pathologie du transport et de l'élimination des complexes immuns par les érythrocytes, avec une application notamment au Lupus erythrémateux disséminé, ou aux infections VIH,
- à la capture des antigènes médiée par les récepteurs Fc en surface des cellules de la lignée monocyte-macrophage,
- à la modulation par des molécules à activité CD16 soluble,
- à la prévention de l'allo-immunisation anti Rh(D) érythrocytaire,
- à l'inhibition de la pénétration cellulaire du virus VIH à l'aide de formes solubles de CD4, d'anticorps dirigés contre les constituants du virus, et/ou de molécules à fonctions enzymatiques.

La présente invention porte sur une protéine multimérique recombinante, caractérisée en ce qu'elle comprend au moins :
a) une molécule de fusion, de nature polypeptidique A constituée d'un fragment C-terminal de la chaîne α de la C4BP compris entre les acides aminés 549 et 124, et d'un fragment polypeptidique hétérologue à ladite chaîne α,
b) une molécule de fusion B, de nature polypeptidique constituée d'un fragment C-terminal de la chaîne β de la C4BP compris entre les acides aminés 235 et 120, et d'un fragment polypeptidique hétérologue à la chaîne β,

Les acides aminés 549 ou 235 représentant respectivement les extrémités C-terminales des molécules de fusion, les fragments hétérologues étant fusionnés par leur extrémité C-terminale au résidu des chaînes α et β respectivement, les molécules en a) et b) étant associées entre elles par leur partie C terminale pour former ladite protéine multimérique.

Préférentiellement, une protéine multimérique recombinante selon l'invention est caractérisée en ce que le fragment C-terminal de la chaîne α est compris entre les acides aminés 549 et 493 et, en ce que le fragment C-terminal de la chaîne β est compris entre les acides aminés 235 et 176. La réassociation des molécules de fusion est réalisée par la formation de ponts disulfures entre les cystéines en position 498 et 510 de l'extrémité C-terminale de la chaîne α, et les cystéines en position 199 et 185 de l'extrémité C-terminale de la chaîne β.

Toute chimère entre les chaînes α et β associant le cas échéant une cystéine de la chaîne α et une cystéine de la chaîne β dans la molécule de fusion A, ou B, ou dans les deux, entre également dans la portée des constructions des protéines multimériques de l'invention.

Notamment, la possibilité de modifier l'espacement entre les deux cystéines peut permettre de modifier le nombre de monomères entrant dans la constitution du multimère.

A titre d'illustration, une augmentation de la distance entre les cystéines peut conduire à une augmentation du nombre de monomères entrant dans le multimère ; en revanche, une diminution de cette distance entrainerait une diminution de ce nombre. Dans certains cas, il peut être avantageux de modifier cette distance de telle façon à modifier la réassociation contrôlée des deux types de chaînes porteuses d'un ligand ou d'un récepteur, quant à leur nombre et à leur proportion.

Dans la présente invention, il a été mis au point un système multimérisant permettant d'obtenir des formules hepta et octamériques à l'aide de l'extrémité C-terminale des chaînes élémentaires de la molécule C4BP. La multimérisation de molécules dont l'extrémité C terminale a été remplacée par la partie C-terminale de la C4BP a ou de la C4BP β, foumit une forme octamérique.

En tout état de cause, le fragment issu de la chaîne β de la C4BP doit être dépourvu des sites de fixation à la protéine S, lesquels sites se situent au niveau des deux SCR de la partie proximale de l'extrémité N-terminale de la chaîne β.

Ainsi, une protéine multimérique recombinante selon l'invention est caractérisée en ce que le rapport en nombre de monomères α / β varie entre 7/1 et 5/3, et est de préférence de 7/1 quand les fragments des parties C-terminales ont une provenance homogène dans les fragments A et B ci-dessus.

Idéalement, la molécule recombinante multimérique est une protéine n'associant que des constituants humains, évitant ainsi toute immunisation xénogénique, et n'activant pas le complément sauf fonctions spécifiques ajoutées intentionnellement dans ce sens. Une telle molécule ne doit pas interagir avec des récepteurs cellulaires ou des molécules plasmatiques et assurer ainsi une meilleure activité à la molécule chimérique par sa multivalence, ainsi qu'une plus longue durée de vie.

Une protéine multimérique selon la présente invention présente des propriétés relatives à l'immuno-intervention, notamment une capacité à moduler l'activité du complément aux fins de générer une opsonisation de manière artificielle. C'est par le caractère hétérofonctionnel que l'on peut attribuer à cette proteine, grâce à l'apport de fragments N-terminaux hétérologues, que ce résultat est obtenu.

A ces fins, une protéine recombinante conforme à l'invention est caractérisée en ce que les fragments hétérologues en A et en B sont issus de ligands spécifiques du système immunitaire, notamment de protéines lymphocytaires de surface du type CD, des anticorps ou de fragments d'anticorps, des antigènes ou de fragments d'antigènes ; les fragments hétérologues en A et B peuvent, selon l'application que l'on souhaite donner à la protéine recombinante, être choisis, sans caractère exhaustif, parmi les polypeptides à activité de ligands suivants :
i) - des fragments issus de protéines lymphocytaires sont le CD4, le CD8, le CD16, le CD35 (ou CR1),
ii) - des anticorps ou fragments d'anticorps ayant une spécificité anti-erythrocytaire et notamment anti Rh(D),
iii) - des antigènes, notamment des antigènes vaccinants, comme le pre-S2 du virus de l'hépatite B,
iv) - une enzyme à visée thérapeutique ou plus particulièrement le fragment de cette enzyme correspondant au site actif dudit enzyme, qui peut être fusionné avec la partie C-terminale de la chaîne alpha pour former le fragment A, le fragment B pouvant alors être constitué par tout type de polypeptide tel que cité en i) à iii).
   Encore plus particulièrement, des combinaisons de A et B particulièrement avantageuses pour mettre en oeuvre l'immuno-intervention de l'invention peuvent être des protéines multimériques dans lesquelles les fragments polypeptidiques de fusion contiennent :
v) - en A le CD4 ou, un dérivé du CD4 et,
   - en B le sc Fv d'un anti Rh(D) ou d'autres anticorps notamment des anticorps neutralisants, ou des cibles antigéniques.

Une autre construction avantageuse est une protéine multimérique dans laquelle les fragments polypeptidiques de fusion contiennent :
- en A un antigène notamment un antigène vaccinant ou une enzyme thérapeutique ou un CD35 (ou CR1) ou un anticorps, ou tout fragment de ceux-ci possédant la propriété de ligand de la molécule entière ;
- en B un anticorps ou un fragment de celui-ci ayant conservé son paratope ;

D'autres protéines multimériques recombinantes avantageuses sont celles dans lesquelles les fragments polypeptidiques de fusion contiennent :
- en A un immogène vaccinant et
- en B un CD4 ou une molécule dérivée pourvu qu'elle conserve la propriété du ligand de la molécule entière.

En outre, la présente invention vise les cellules eucaryotes ou procaryotes transfectées par un ou plusieurs plasmides contenant une séquence d'acide nucléique hétérologue et codant au moins pour une molécule polypeptidique de fusion A et une molécule polypeptidique de fusion B.

Les différentes lignées cellulaires qui peuvent être utilisées pour être transfectées par des plasmides sont de préférence des cellules eucaryotes susceptibles de réaliser la glycosylation post-traductionnelle ; on peut citer à titre d'exemple, des levures ou des cellules de lignées animales telles des cellules de types fibroblastiques, comme les BHK ou les CHO, ou encore des lignées lymphocytaires comme des lymphocytes immortalisés.

Selon différentes réalisations de la présente invention, les cellules peuvent être :
- co-transfectées; par deux plasmides distincts, ou
- transfectées par un plasmide codant pour un premier polypeptide puis sur-transfectées par le deuxième plasmide codant pour le deuxième polypeptide
ou,
- résultent de la fusion de deux cellules dont l'une a été transfectée par le Plasmide codant pour le premier polypeptide et l'autre a été transfectée par un plasmide codant pour le deuxième poplypeptide.

Les fusions de cellules sont réalisées par des méthodes classiques, soit par action du PEG (polyethylène glycol), ou encore par action du virus Epstein-Barr, ou toute autre méthode classique pour fusionner deux cellules eucaryotes différentes.

Plus particulièrement, lesdites cellules peuvent être transfectées par le premier plasmide qui est celui déposé le 12 juillet 1995 à la C.N.C.M. sous le N° 1-1610 et par le deuxième plasmide qui est celui déposé à la

C.N.C.M; le 12 juillet 1995 sous le N° 1-1611.

La présente invention concerne également un procédé de préparation d'une protéine multimérique selon l'invention. Ce procédé est caractérisé en ce qu'il comprend au moins les étapes suivantes :
- la transfection de lignées cellulaires cibles par au moins un plasmide contenant une séquence hétérologue codant pour une chaîne A ou B, telles que définies ci-dessus,
- l'expression et l'isolement des chaînes hétérologues A ou B,
- la mise en milieu oxydant desdits polypeptides, dans des proportions déterminées,
- l'isolement des multimères.

De préférence, ce procédé de préparation est caractérisé en ce que les lignées transfectées ont été soit :
- co-transfectées par deux plasmides porteurs de séquences d'ADN codant respectivement pour les polypeptides A et B, ou
- transfectées par un plasmide codant pour un premier polypeptide puis sur-transfectées par le deuxième plasmide codant pour le deuxième polypeptide, ou ,
- résultent de la fusion de deux cellules dont l'une a été transfectée par le plasmide codant pour le premier polypeptide et l'autre a été transfectée par un plasmide codant pour le deuxième poplypeptide.

Par ailleurs, la présente invention porte sur l'utilisation d'une protéine recombinante telle que précédemment définie, dans la fabrication d'un médicament, et plus particulièrement, d'un médicament destiné à
- la prévention de l'allo-immunisation foeto-matemelle ou,
- la thérapeutique, ou la prophylaxie des infections virales, bactériennes
ou parasitaires,
- la thérapeutique des maladies auto-immunes et notamment le lupus erythrémateux disséminé, ou les maladies allo-immunes.

De façon plus générale, la présente invention porte sur l'utilisation d'une protéine recombinante telle que précédemment définie dans la fabrication d'un médicament permettant, selon la fonctionnalité attribuée aux ligands, ou aux récepteurs, une immuno-intervention notamment dans l'opsonisation ou la non-opsonisation de cellules cibles, par activation, modulation ou inhibition du complément.

L'homme du métier saura, au fur et à mesure de la découverte des fonctionnalités de certaines protéines ou de certains ligands ou récepteurs, construire une protéine multimérique recombinante selon l'invention la mieux adaptée à l'effet recherché.

Avantageusement, l'utilisation de la protéine multimérique selon l'invention est caractérisée en ce qu'elle permet une activation du complément suffisante à provoquer l'opsonisation de cellules dont les sites antigéniques ou épitopiques ne sont pas naturellement susceptibles de déclencher une telle activation.

Entre également dans le cadre de la présente invention une composition pharmaceutique caractérisée en ce qu'elle comprend en tant que principe actif une protéine multimérique recombinante telle que décrite ci-dessus. Ladite composition pharmaceutique peut permettre une immuno-thérapie ou une immuno-prévention de différentes pathologies notamment celles liées aux infections virales, bactériennes, aux maladies auto-immunes ou allo-immunes.

Une protéine recombinante selon l'invention est également utilisable dans un test de diagnostic nécessitant l'intervention d'au moins deux ligands ou récepteurs différents.

La faisabilité du multimère de très haut poids moléculaire a été vérifiée dans un modèle de multi CR1 (env. 1,5 million daltons). Cette molécule est fonctionnelle et inhibe l'activation du complément dans un modèle de lyse dépendante du complément, d'erythrocytes recouverts d'anticorps, à des concentrations inférieures à celles requises pour le CR1 soluble monomérique.

La faisabilité d'hétéro-chimères associant différentes fonctions a été établie ensuite en utilisant, d'une part l'anticorps anti Rh(D), et plus particulièrement la partie variable Fv de cet anticorps et plus particulièrement encore la partie simple chaîne de cette partie variable appelée scFv, dans le cas présent associée à la molécule CD35 ou (CR1) susceptible d'inhiber ou de moduler l'activité du complément ; l'autre système utilisé est un système hétéro-multimérique de type CD4/antigène.

Les exemples qui suivent, n'ont aucun caractère limitatif et ne servent qu'à démontrer la faisabilité des constructions de ces hétéro-multimères recombinants à des fins d'immuno-intervention ; les figures qui illustrent les exemples ont les significations suivantes :
- la figure 1 représente un vecteur d'expression constitué d'un plasmide contenant la séquence codant pour le CD4 et appelé sT4CD4-C4 BP ;
- la figure 2 représente un vecteur plasmidique contenant la séquence codant pour le CD16 multimérique .
- la figure 3 représente un vecteur plasmidique contenant la séquence codant pour le CR1 multimérique ;
- la figure 4 représente un autre vecteur plasmidique, pCDM8, codant pour le même CR1 multimérique ;
- la figure 5 représente un vecteur plasmidique contenant la séquence codant pour le scFv de l'anticorps anti Rh(D) multimérique ;
- la figure 6 représente un autre vecteur plasmidique, pST4, contenant la même séquence codant pour le scFv de l'anticorps anti Rh(D) ;
- la figure 7 représente un troisième vecteur plasmidique contenant cette même séquence codant pour le scFv de l'anticorps anti Rh(D) ;
- la figure 8 représente un vecteur plasmidique de type pKC3B contenant la séquence codant pour le CR1 multimérique.
   Dans toutes ces figures, les enzymes de restriction permettant l'insertion de la séquence hétérologue sont indiqués par leur nomenclature classique.
- la figure 9 représente le résultat obtenu avec le scFv multimérique sur l'agglutination des globules rouges présentant ou ne présentant pas l'antigène Rhésus. Le tube en A représente le témoin positif dans lequel
   les globules rouges O Rh+ sont agglutinées par un anticorps monoclonal anti R(h) natif ; le tube B représente le témoin négatif dans lequel des globules rouges O Rh- ne sont pas agglutinées par le scFv anti R(h) des multimériques ; le tube C représente l'essai dans lequel des globules rouges O Rh+ et scFv anti Rh+ sont agglutinées ; le tube D est un autre témoin négatif dans lequel des globules rouges O Rh- sont mis en présence d'un milieu de culture sans anticorps.

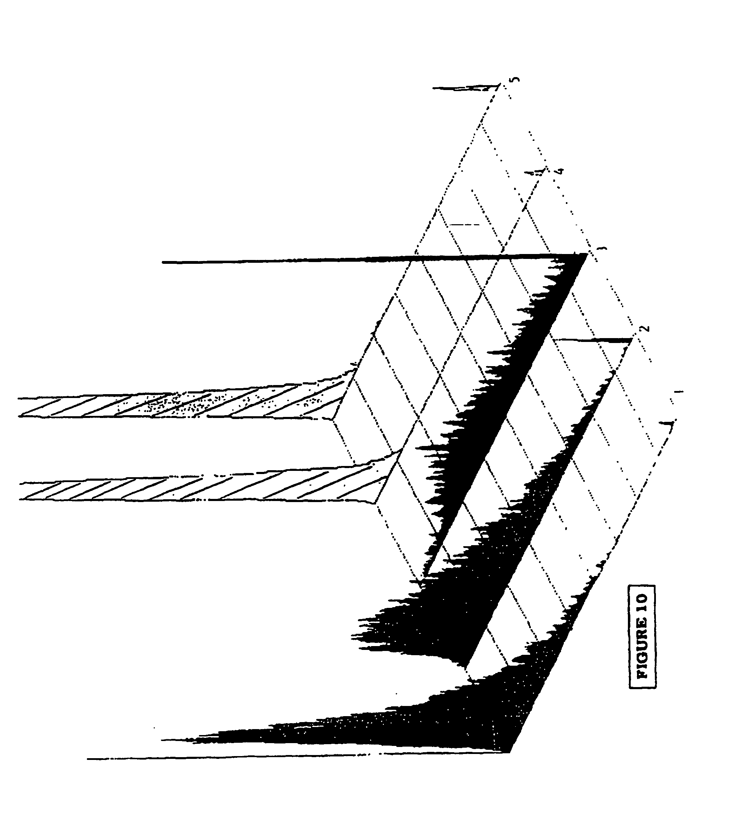

- la figure 10 représente le profil obtenu en cytométrie de flux après fixation erythrocytaire d'une hétéro-chimère anti Rh(D) -CR1.

Cinq pistes sont représentées dans lesquelles :
- la première piste représente des globules rouges O Rh+ non papaïnés avec une densité de CR1 de 180 sites ;
- la deuxième piste représente des glolules rouges O Rh+ non papaïnés avec une densité de CR1 de 550 sites ;
- la troisième piste représente des globules rouges O Rh+ papaïnés, qui ont perdu leur densité de 180 sites de CR1 ; ces erythrocytes reconstitués en CR1 à l'aide de l'hétéro-chimère scFv anti Rh+/CR1 expriment la densité supra physiologique de 1 200 sites CR1 par erythrocyte ;
- les pistes 4 et 5 représentent des témoins dans lesquels pour la piste 4, sont des globules rouges O Rh+ papaïnés, et pour la piste 5, sont des globules rouges O Rh- papaïnés traités avec l'hétéro-chimère scFv anti Rh(D)/CR1.

### I - Construction des chaînes CR1 - C4 BP

L'avantage d'utiliser le CR1 dans une construction multimérique selon l'invention résulte des travaux des inventeurs sur le devenir physiologique du CR1 chez le sujet normal. Ils ont pu ainsi déterminer les paramètres d'un catabolisme physiologique du CR1 érythrocytaire et ses relations avec le polymorphisme génétique de densité de CR1 érythrocytaire. Ils ont pu également préciser le catabolisme du CR1 érythrocytaire chez les patients lupiques, c'est-à-dire souffrant d'un lupus erythrémateux disséminé. La distribution parmi les patients lupiques et les sujets normaux des différents génotypes du polymorphisme de longueur et de nombre de sites de liaison C3b/C4b de CR1 a également été étudiée. Des molécules de CR1 recombinantes permettant de modifier la densité de CR1 érythrocytaire pour restaurer leur état physiologique ou réaliser des érythrocytes "armés" de densités "supra-physiologiques" de CR1 ont ensuite été préparées. Le potentiel du CR1 soluble a été démontré dans différents modèles en particulier d'ischémie myocardique expérimentale et de phénomène d'Arthus. Une molécule de CR1 soluble multimérique est produite, et son pouvoir anti-inflammatoire, sa durée de vie plasmatique ainsi que son espace de distribution sont étudiés chez l'animal. Un couplage chimique aux érythrocytes de monomères réduits par leur groupement SH libre est réalisée. Des érythrocytes sont ainsi armés de densités supraphysiologiques de CR1 présentés de façon fonctionnelle, et leur capacité de liaison de complexes immuns artificiels antigène Hbs/anticorps antiHBs opsonisés par du C3b est alors étudiée.

Les résultats obtenus avec un anti Rh(D) sont montrés dans l'exemple I ci-après.

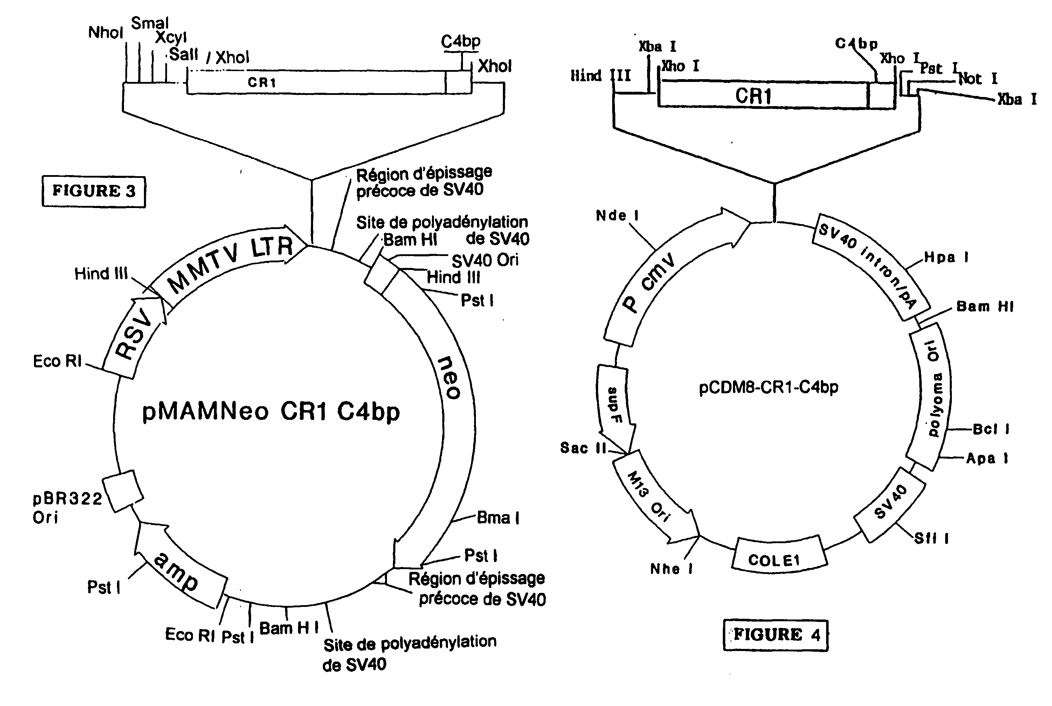

Les constructions utilisées pour réaliser la transfection C4 BP - CR1 sont représentées dans les figures 3,4 et 8 dans les plasmides pMAMneo, pCDM8 et pKC3b.

### a) Construction de pMAMneo CR1-C4BP :

L'ADN complémentaire codant pour le CR1 avait été inséré aux sites *Xho I* et *Not I* dans le plasmide pCDM8 (dû à l'obligeance de T. J. Bartow, D. T.

Fearon et W. Wong, John Hopkins Hospital, Baltimore, U.S.A.).

La séquence codant pour la partie extra-membranaire du CR1 est extraite par digestion de ce plasmide par les enzymes de restriction *Xho I* et *Bal I*.

La partie C terminale de la C4BP est amplifiée par les amorces 5'-GAGACCCCCGAAGGCTGTGA-3', et 5'- CTCGAGTTATAGTTCTTTATCCCAAGTGG-3', cette deuxième amorce contenant un codon stop et un site de restriction *Xho I*.

Les séquences codant pour la partie C terminale de la C4BP et pour la partie extra-membranaire du CR1 sont insérées dans pMAM Neo (Clontech, Palo Alto, USA) au site *Xho I* (figure 3) CR1-C4BP par digestion par *Xho I*, et insérée dans le plasmide pCDM8 (Invitrogen, San Diego, USA).

### b) Construction pCDM8 CR1-C4BP :

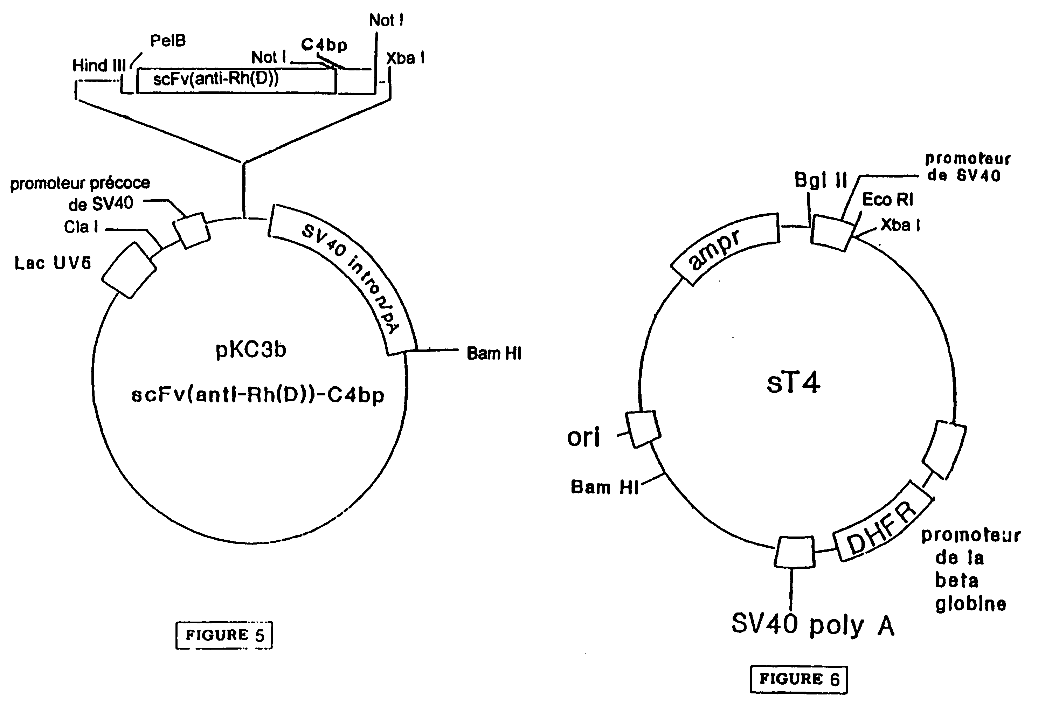

La séquence codant pour la protéine de fusion CR1-C4BP a été extraite de pMAM Neo CR1-C4BP par digestion par *Xho I*, et insérée dans le plasmide pCDM8 (Invitrogen, San Diego, USA) (figure 4).

### c) Construction pKC3b CR1-C4BP :

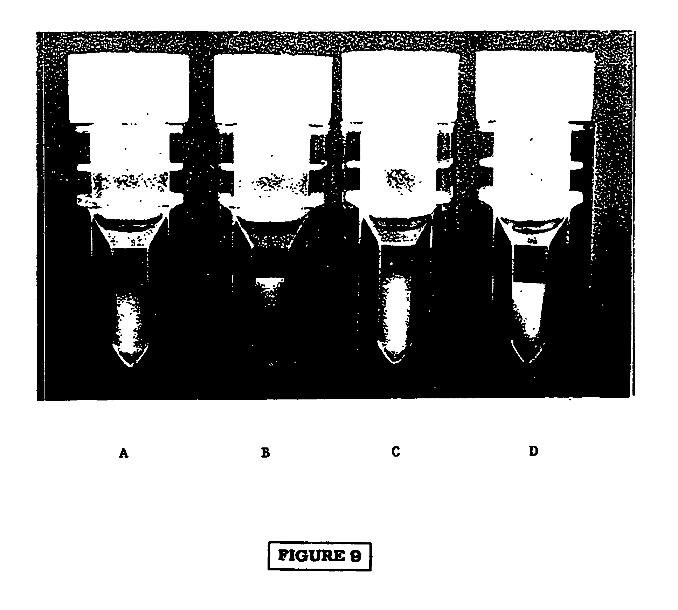

La séquence codant pour la protéine de fusion CR1-C4BP a été extraite de pMAM Neo CR1-C4BP par digestion par *Xho I*, et insérée dans le plasmide pKC3b (figure 8).

### II - Construction du multimère recombinant :

Un fragment C-terminal de la chaîne α de la C4BP a été recopié par PCR à partir de DNA génomique. Il figure dans un seul exon. La taille minimale se situe au-delà de la deuxième cystéine en partant de l'extrémité C-terminale, la taille optimale se situe quelques acides aminés au-delà, réalisant un espaceur de 5 à 10 acides aminés, soit au total 58 AA.

La taille maximale choisie est de 6 SCR pour éviter le site de liaison C3b-C4b. Ce fragment maximal est synthétisé à partir d'un cDNA du mRNA de la C4 BP, puisqu'il comporte plusieurs exons. Le fragment optimal de la partie C-terminale de la C4 BP est recopié à nouveau par PCR à l'aide d'amorces pourvues à leurs extrémités de bras comportant les sites de restriction enzymatique adéquats pour un montage dans un vecteur donné comportant déjà le gène de la protéine que l'on souhaite multimériser. Un site enzymatique proche de la partie C-terminale de cette protéine, ou situé dans sa partie extra-membranaire, est choisi permettant l'insertion en 3' du fragment multimérisant.

L'extrémité 3' du fragment multimérisant est liée soit à un site du vecteur, soit à un site situé au-delà dans le gène de la protéine d'intérêt. La partie du gène de la protéine d'intérêt située en 3' du fragment multimérisant n'est de toute façon plus traduite, puisque le fragment multimérisant comporte un codon stop.

Il est donc possible ainsi de modifier très facilement un vecteur d'expression contenant le gène d'une protéine donnée par la simple insertion du fragment, sans autre modification.

Les vecteurs pCDM8, ST4, pMAMneo, ont été utilisés pour les différents exemples d'application du système multimérique selon l'invention.

L'homme du métier saura toujours trouver les vecteurs existants aujourd'hui ou qui risquent d'être mis au point, susceptibles de présenter la meilleure efficacité pour transfecter la protéine de fusion dans les cellules et la faire exprimer.

### Exemple d'application N° 1 :

### Prévention de l'alloimmunisation antiRh(D).

Dans le cadre d'une prévention de l'alloimmunisation anti Rh(D), des molécules hétéro-multimériques associant des fonctions érythrocytaires et CR1 sont produites. Elles permettront une liaison aisée de CR1 à des érythrocytes assurant des densités de CR1 parfaitement maitrisables.

La molécule d'anticorps utilisée pour produire le scFv anti Rh(D) a été produite et séquencée dans le laboratoire de Philippe ROUGER à l'Institut National de Transfusion Sanguine (INTS) (9).

### Construction des vecteurs comportant la séquence codant pour le scFv anti Rh(D) et la partie terminale de la chaîne α de la C4BP.

Dans un premier temps, un site épitopique d'un anticorps anti-Rhésus a été réduit en une structure de type scFv (pour single chaine Fv) pour une expression dans E. Coli par transfection par un vecteur phagique.

Les constructions de type scFv sont des fragments d'anticorps représentant la partie variable de l'anticorps et ne contenant qu'une seule chaîne. Cette technique a été décrite par G. WINTER (10). La séquence codante pour ce scFv a ensuite été transférée dans un vecteur d'expression après addition du système multimérisant.

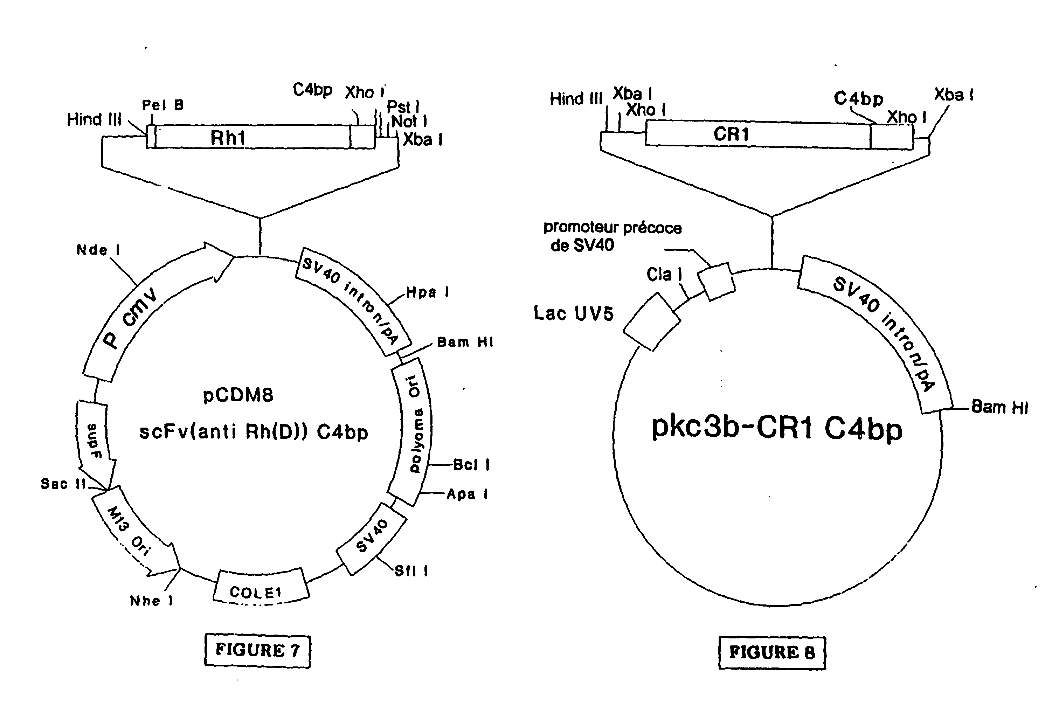

Nous avons décrit plus haut la construction des vecteurs d'expressions porteurs de la séquence codant pour le scFv de l'anticorps anti Rh(D), et qui sont représentés sur les figures 6 et 7.

La partie C terminale de la C4BP a été amplifiée par les amorces 5'-GCGGCCGCAGAGACCCCCGAAGGCTGTG-3' contenant un site de restriction *Not I* et 5'-CCACTTTGGATAAAGAACTATAA-3' contenant un site de restriction *Xho I*.

Ce fragment a été inséré au site *Not I* en 3' du gène scFv anti Rh(D). Cette séquence a ensuite été insérée dans le plasmide pCDM8 et insérée aux sites *Hind III* et *Xho I* de pKC3b. Cette construction est représentée dans la figure 5.

La figure 5 représente une autre construction du scFv du anti Rh(D) C4BP dans le plasmide pKC 3B.

Les plasmides sont ensuite utilisés pour transfecter des cellules animales et notamment des cellules CHO DHFR⁻.

Les cellules CHO-DHFR⁻ (American Type Culture Collection, Rockville, USA) ont été transfectées selon la technique au phosphate de calcium (Calcium phosphate transfection kit, 5 prime 3 prime Inc., Boulder, U.S.A).

Les cellules sont cultivées en milieu HAM déficient en Hypoxanthine et Thymidine (Biochrom, Vindelle, France) avec 10 % de sérum de veau dyalisé (SVF GIBCO BRL, Paisley, Ecosse) et 1% de glutamine (Sigma, St Louis, USA).

La fonctionnalité des protéines multimériques reconstituées, soit C4 BP-scFv, soit C4 BP-Rh(D)/CR1 a été étudiée.

La production fonctionnelle de scFv multimérique a été réalisée, telle que démontrée (i) par un marquage biosynthétique et une immunoprécipitation suivis d'analyse en PAGE SDS, (ii) par la détection en cytométrie de flux de multi scFv anti Rh(D) fixés sur des érythrocytes, (iii) par le caractère agglutinant en faible force ionique sur des globules papaïnés de chimères multi scFv anti Rh(D), (iiii) par analyse d'interactions moléculaires au moyen d'un appareil de détection d'ondes évanescentes (IASys FISONS) vérifiant la fixation sur des érythrocytes de la chimère multimérique anti scFv anti Rh(D).

Par la suite, la faisabilité d'hétéromultimères associant différentes fonctions a été établie par la réalisation d'une chimère anti R(h)ésus D/CR1. Son caractère fonctionnel a été démontré en cytométrie de flux.

Les résultats obtenus en cytométrie de flux sont représentés sur la figure 10. Il est clair sur cette figure que si l'on compare les pistes 3 et 5 dans lesquelles respectivement les gobules rouges sont Rh+ ou Rh-, on observe que seules les globules rouges possédant l'antigène de surface sont agglutinées. Ce que cette figure permet également de voir, c'est que ce n'est pas l'effet de la papaïne qui permet cette agglutination puisque les globules rouges Rh+ papaïnées ne sont pas agglutinées par le CR1.

Il a été en effet possible de fixer une densité supraphysiologique de molécules CR1 sur des érythrocytes préalablement papaïnés et donc déplétés en CR1, par la fixation sur les molécules rhésus D d'une chimère mixte multimérique anti Rh(D) /CR1.

### Exemple d'application N° 2 : Destruction extracellulaire du VIH

Si la réponse immune humorale est impuissante à éradiquer le virus VIH, elle est cependant efficace contre de nombreux agents infectieux contre lesquels des anticorps neutralisant sont régulièrement produits par les sujets vaccinés.

Des anticorps naturels peuvent conférer une protection contre de nombreux agents infectieux bactériens ou viraux, infectant d'autres espèces.

Certains motifs antigéniques ont été caractérisés comme cibles de ce type d'anticorps pour leur rôle dans le rejet suraigu vasculaire de transplantation xénogéniques.

Le rôle potentiellement défavorable de la réponse immune humorale et de l'activation du complément vis-à-vis de l'infection par virus VIH a été démontré.

Dans certaines circonstances, l'opsonisation des virions peut conduire à faciliter l'ingestion macrophagique ou la liaison lymphocytaire des virions par l'intermédiaire de récepteurs cellulaires pour le complément ou le fragment Fc des IgG. En revanche le virus VIH, en tant que virus enveloppé, est extrêmement sensible à l'action lytique du complément lorsque l'activation de ce dernier est suffisante pour initier sa voie finale lytique ainsi que la fixation de son complexe d'attaque membranaire. Les rétrovirus xénogènes sont détruits extrêmement efficacement par le sérum humain normal du fait de l'existence d'anticorps naturels, par l'intermédiaire d'une activation du complément. Ce phénomène avait même fait conclure dans les années 1970 à l'immunité naturelle de l'espèce humaine vis-à-vis des rétrovirus.

Le but recherché est le détournement d'une réponse immune humorale lytique vers les virions VIH, l'attachement aux virions étant réalisé par la composante CD4 d'une protéine recombinante hétéromultimérique. Pour cela, les inventeurs ont pris en compte le fait que la très grande variabilité du virus VIH est cependant limitée par le maintien constant, essentiel pour le virus, d'une capacité de liaison à CD4, pour sa pénétration cellulaire.

Réciproquement, la molécule CD4 est capable de se lier à l'ensemble des virions VIH, au contraire de nombreux anticorps neutralisants au spectre d'efficacité restreint à un petit nombre de sous-type. La molécule de CD4 soluble inhibe l'infection cellulaire par le VIH. Cependant, les concentrations nécessaires à son action, particulièrement pour la neutralisation d'isolats sauvages, rendent impraticable son emploi clinique. Différentes tentatives ont été faites pour améliorer sa demi-vie, son avidité et/ou apporter une fonction Fc gamma effectrice de liaison cellulaire ou d'activation de complément par des constructions de type : CD4/IgG bi ou tétravalent.

Les inventeurs ont développé une molécule CD4 multivalente heptamérique à l'aide d'un système multimérisant C4BP, dont l'efficacité biologique in vitro a été démontrée par l'inhibition de l'infection de cellules sensibles par le VIH à des concentrations inhibitrices usuelles de CD4 soluble monomérique.

Dans le présent exemple, les inventeurs ont, plutôt que d'inhiber la pénétration cellulaire du virus, cherché à obtenir sa destruction extracellulaire à l'aide de molécules solubles capables, d'une part de se lier sur le virion, et d'autre part d'apporter une fonction effectrice antigène, élicitant la destruction du virus par une réponse anticorps dépendante du complément préexistante chez l'individu. Cette réponse est dirigée contre un antigène sans rapport avec le VIH, mais vis-à-vis duquel une efficacité neutralisante et lytique du système immunitaire a été néanmoins testée.

En d'autres termes, le virus VIH ayant la capacité de se « déguiser », de se « cacher » ou d'« agiter des leurres », les inventeurs ont cherché à l'orner de cibles que le système immunitaire de l'individu sait identifier et traiter efficacement. Pour ce faire, deux constructions plasmidiques ont été réalisées portant respectivement le CD4 ou le fragment du CD4 porteur de la fraction «ligand » et un antigène.

### a) Construction du vecteur ST4 CD4-C4BP

Les 183 derniers nucléotides de la séquence codant pour la C4BP ont amplifiés par PCR sur de l'ADN génomique, en utilisant les amorces : 5'-GAGACCCCCGAAGGCTGTGTGA-3' et 5'ATTTCTAGAGAGTTATAGTTCTTTATCCAAAGTGGA-3', cette dernière amorce contenant un codon stop et un site de restriction pour Xba I. Ce fragment de PCR a été lié en 5' à une séquence oligonucléotidique synthétique double-brins:
5'CCGGGACAGGTCCTGCTGGAATCCAACATCAAGGTTCTGCCCACAG-3'. Ce fragment codant pour l'extrémité C-terminale de la partie extramembranaire de CD4 et ayant un site Ava I en 5'.

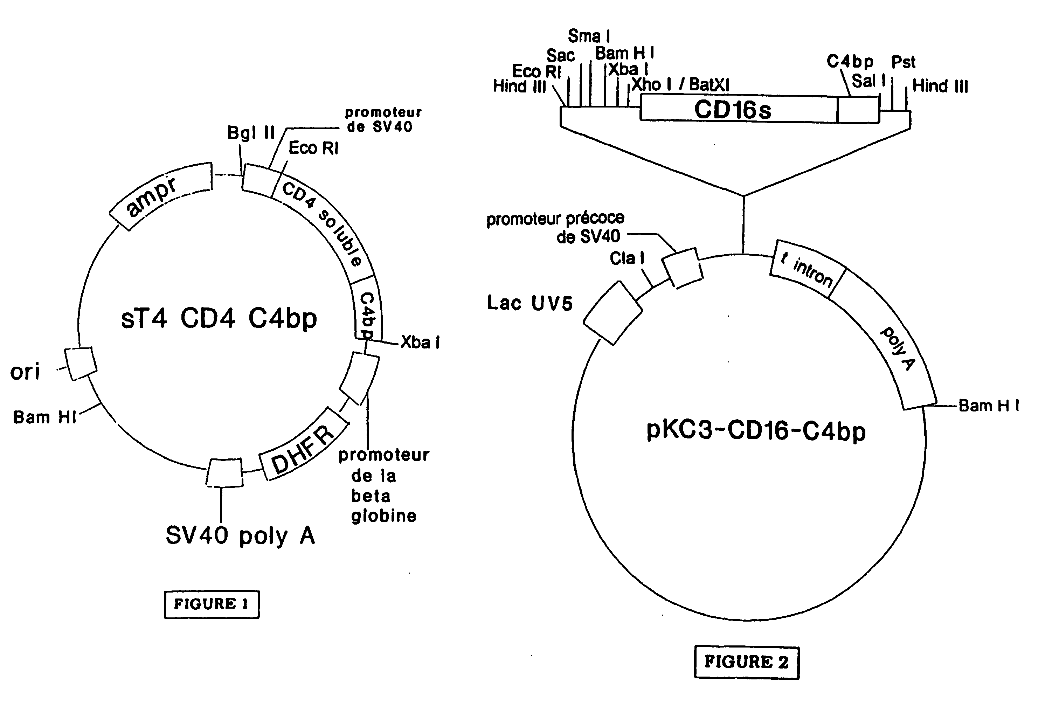

Cette séquence a été insérée aux sites Ava I et Xba I dans le plasmide sT4 CD4 contenant la construction codant pour le CD4 soluble et cette construction est représentée sur la figure 1.

### b) Construction des molécules de fusion C4BP- antigènes

Dans un premier temps il s'agit de rechercher quels paramètres conduisent la réponse anticorps gp 120 des sujets infectés à une activation du complément jusqu'à la boucle d'amplification du C3, aboutissant à une opsonisation plutôt favorable à l'individu, sans s'accompagner pour autant de l'activation de la voie finale commune qui aboutirait à la lyse du virion. Le rôle de molécules de surface d'inhibition de l'activation du complément que le virion a emporté de la surface cellulaire, est démontré. Le relargage de particules d'enveloppe lors de la fixation d'anticorps (shedding) est également défavorable à l'activation terminale du complément. L'activation de la voie finale commune du complément nécessite une densité critique d'activation du C3 pour initier la conversion de C5. Celle-ci n'est pas réalisée par la fixation d'IgG sur des épitopes de la gp 120 trop éloignés les uns des autres.

L'apport d'une « *grappe* » d'antigènes grâce aux constructions de l'invention pour chaque site de liaison sur le virion permet donc de déclencher une activation locale suffisante du complément.

Différentes catégories d'antigènes ont été considérées : des antigènes vaccinaux, des antigènes bactériens contre lesquels l'espèce humaine est universellement immunisée, des antigènes xénogéniques cibles d'anticorps naturels.
- des antigènes vaccinaux existant sous forme de gènes clonés codant pour une protéine exprimable en cellules eucaryotes (antigène Hbs, anatoxine tétanique...),
- des antigènes bactériens vis-à-vis desquels il existe une forte immunité dans l'espèce humaine (Escherichia-Coli, Klebsielle, flagelline de Shigelle, ou antigène de Salmonelle),
- des molécules possédant les séquences protéiques acceptrices de glycosylations xénogéniques peuvent également être envisagées après qu'elles aient été produites par des cellules animales possédant de fortes activités glycosyl transférase qui leur grefferont les cibles glucidiques d'anticorps naturels connus comme réagissant fortement en xénotransplantation (par exemple : groupement alpha-galactosyl, obstacle aux transplantations xénogéniques porc-homme).

Ces mini-anticorps seront utilisés comme agents de liaison érythrocytaire d'hétérochimères les comportant dont ils ne représenteront qu'une valence de type C4BP β associée à une molécule antigénique multimérique de type C4BP α heptamérique. Le système antigénique le plus efficace sera ainsi sélectionné dans un test de criblage aisément quantifiable. Il sera alors transféré dans une chimère recombinante CD4/cible antigénique dont différents ratios CD4/antigène (1CD4/7antigènes ou nCD4/M antigènes) seront testés dans un modèle d'inhibition d'infection virale in vitro. Les antigènes cibles les plus intéressants ont été insérés dans des constructions hétéromultimériques comportant le CD4 et testés pour leur capacité à permettre la destruction de virions VIH en présence de sérum humain et de complément, le pouvoir infectieux résiduel étant évalué dans un test d'inhibition d'infection cellulaire in vitro

### Matériels et méthodes :

Outre les constructions citées plus haut, les techniques de transfection et de culture cellulaires utilisées ont été les suivantes :

### Transfection :

Les cellules CHO-DHFR⁻ (American Type Culture Collection, Rockville, USA) ont été transfectées selon la technique au phosphate de calcium (Calcium phosphate transfection kit, 5 prime 3 prime Inc., Boulder, U.S.A).

### Culture Cellulaire:

Les cellules sont cultivées en milieu HAM déficient en Hypoxanthine et Thymidine (Biochrom, Vindelle, France) avec 10 % de sérum de veau dyalisé (SVF GIBCO BRL, Paisley, Ecosse) et 1% de glutamine (Sigma, St Louis, USA).

Les cellules transfectées par pMAMNeo sont sélectionnées par leur capacité de résistance à la néomycine (G418, 0.7 microg/ml) (Sigma).

La production de mCR1 dans les cellules transfectées par le pMAMneo

CR1-C4BP est induite par la dexamethasone (0.8 microg/ml).

Les inventeurs ont utilisés pour leurs expériences un appareil de cultures cellulaires en continu, en fibres creuses pour la production de protéines recombinantes à l'échelle de quelques milligrammes ou quelques dizaines de milligrammes de protéines recombinantes. La plupart des expériences pourront être menées à partir de surnageants de culture bruts ou concentrés. Des préparations purifiées à petite échelle ont également été préparées.

Les synthèses d'olignonucléotides utilisées pour les constructions vectorielles ont été effectuées pour adapter le fragment C4BP C terminal sur chaque construction. Egalement les séquences nucléotidiques ont été déterminées sur séquenceur automatique à fluorescence pour le contrôle des constructions.

### Commentaires

Les protéines multimériques de l'invention, leur utilisation dans la fabrication d'un médicament à visée prophylactique ou thérapeutique, ou leur utilisation comme outil de diagnostic ou de recherche sont très puissantes.

Leur utilisation peut être un outil efficace pour l'analyse de mécanismes physiologiques dans la réponse immune, de même que pour comprendre la physiopathologie de certaines affections dysimmunitaires.

De telles molécules doivent permettre une immuno-intervention plus sophistiquée ouvrant la possibilité de mieux étudier in vitro de nombreux mécanismes physiopathologiques. Dans certains cas, cette intervention ouvrira la voie à une immuno-manipulation in vivo dans un but thérapeutique. Il s'agit donc à la fois d'outils de recherche clinique physiopathologique, d'une recherche expérimentale in vitro et d'outils thérapeutiques in vivo.

### BIBLIOGRAPHIE

**1.** Matsuguchi T. Okamura S, Aso T. Niho Y. Molecular cloning of the cDNA coding for PRP : identity of PRP as C4BP. Biochem Biophys Res Commun 1989 ; 1 : 139-44.
**2.** Scharfstein J. Ferreira A. Gigli I, Nussenzweig V. Human C4-binding protein. I. Isolation and characterization. J exp Med 1978 ; 148 : 207-22.
**3.** Fujita T, Gigli I, Nussenzweig V. Human C4-binding protein II. Role in proteolysis of C4b by C3b-inactivator. J. Exp Med 1978; 148 : 1044-51.
**4.** Chung LP, Bentley DR, Reid KBM. Molecular cloning and charaterization of the cDNA coding for C4b-binding protein, a regulatory protein of the classical pathway of the human comlement system. Biochemistry 1985 : 230 : 133-41.
**5**. Monte G.Thrombosis and Haemostasis - 69 (1) 86 (1993).
**6.** Hillarp. A (1990) PNAS vol 87 pp 1183-1187.
**7.** Hillarp. A (1991) Scand. J. Chin. Lab. Invest. Fi, Suppl 204 : 57-69.
**8.** Fanger M.W. Immunomethods 1994 - P. 72 à 81 « Production and use of anti FcR bi-specific antibodies ».
**9.** Goossens D., Champomier F, Rouger Ph. and Salmon Ch. Human monoclonal antibodies against blood group antigens : preparation of a series of stable EBV Immortalized B clones producing high levels of antibody of different isotypes and specifites. J. of immonological methods., 101, 193,1987.
**10**. Winter G. Nature 1990 -348- P. 552-554. M. Cafferty J., Griffiths A, Winter G., Chiswell. « Phage antibodies filamentous phase displaying antibody variable domains ».

## Revendications

1. Protéine multimérique recombinante permettant une immuno-intervention en pathologie dysimmunitaire humaine, **caractérisée en ce qu'**elle comprend au moins :
a) un monomère de fusion A, de nature polypeptidique constituée d'un fragment C-terminal de la chaîne α de la C4BP compris entre les acides aminés 124 et 549 formant des ponts disulfures avec l'extrémité C-terminale de la chaîne β de la C4BP et d'un fragment polypeptidique hétérologue à ladite chaîne α,
b) un monomère de fusion B, de nature polypeptidique constituée d'un fragment C-terminal de la chaîne β de la C4BP compris entre les acides aminés 120 et 235 formant des ponts disulfures avec l'extrémité C-terminale de la chaîne α de la C4BP et d'un fragment polypeptidique hétérologue à la chaîne β,
les molécules en a) et b) étant associées dans leur partie C-terminale pour former ladite protéine multimérique.

2. Protéine multimérique recombinante selon la revendication 1, **caractérisée en ce que** le fragment C-terminal de la chaîne α est compris entre les acides aminés 493 et 549 et, **en ce que** le fragment C-terminal de la chaîne β est compris entre les acides aminés 176 et 235.

3. Protéine multimérique recombinante selon la revendication 1 ou 2, **caractérisée en ce que** le rapport en nombre de monomère α / β varie entre 7/1 et 5/3, et est de préférence de 7/1.

4. Protéine multimérique recombinante selon l'une des revendications 1 à 3 **caractérisée en ce que** les fragments hétérologues en A et en B sont issus de ligands spécifiques du système immunitaire, notamment issus de protéines lymphocytaires de surface du type CD, d'anticorps ou de fragments d'anticorps, d'antigènes ou de fragments d'antigènes.

5. Protéine multimérique recombinante selon la revendication 4, **caractérisée en ce que** les fragments issus de protéines lymphocytaires sont le CD4, le CD8, le CD16, le CD35 (ou CR1).

6. Protéine multimérique recombinante selon la revendication 4, **caractérisée en ce que** les anticorps ou fragments d'anticorps ont une spécificité anti Rh(D).

7. Protéine multimérique recombinante selon la revendication 4, **caractérisée en ce que** les antigènes sont des antigènes vaccinants.

8. Protéine multimérique recombinante selon l'une des revendications 1 à 3, **caractérisée en ce que** le fragment hétérologue en A est une enzyme thérapeutique.

9. Protéine multimérique recombinante selon l'une des revendications 1 à 3, **caractérisée en ce que** les fragments polypeptidiques hétérologues contiennent :
- en A le CD4 ou un dérivé du CD4 et;
- en B le scFv d'un anticorps, notamment un anticorps neutralisant ou, un anti Rh (D).

10. Protéine multimérique recombinante selon l'une des revendications 1 à 3, **caractérisée en ce que** les fragments polypeptidiques hétérologues contiennent :
- en A un antigène, notamment un antigène vaccinant, ou une enzyme thérapeutique ou un CD35 (ou CR1) ou un anticorps, ou tout fragment de ceux-ci possédant la propriété de ligand de la molécule entière,
- en B un anticorps ou un fragment de celui-ci ayant conservé son épitope.

11. Protéine multimérique recombinante selon l'une des revendications 1 à 3, **caractérisée en ce que** les fragments polypeptidiques hétérologues contiennent :
- en A un immunogène vaccinant et
- en B un CD4 ou une molécule dérivée pourvu qu'elle conserve la propriété du ligand de la molécule entière.

12. Cellules procaryotes ou eucaryotes **caractérisées en ce qu'**elles ont été transfectées par un ou plusieurs plasmides contenant une séquence d'acide nucléique hétérologue codant pour au moins une molécule polypeptidique de fusion A et une molécule polypeptidique de fusion B selon la revendication 1.

13. Cellules selon la revendication 12 **caractérisées en ce que** les cellules ont été soit,
- co-transfectées par deux plasmides distincts, soit
- transfectées par un premier plasmide codant pour un premier polypeptide puis sur-transfectées par le deuxième plasmide codant pour le deuxième polypeptide, soit,
- résultent de la fusion de deux cellules dont l'une a été transfectée par un plasmide codant pour le premier polypeptide et l'autre a été transfectée par un plasmide codant pour le deuxième polypeptide.

14. Cellules selon l'une des revendications 12 ou 13 **caractérisées en ce que** le premier plasmide est celui déposé le 12 juillet 1995 à la C.N.C.M. sous le N° I-1610 et le deuxième plasmide est celui déposé à la C.N.C.M., le 12 juillet 1995 sous le N° I-1611.

15. Procédé de préparation d'une protéine multimérique définie selon l'une quelconque des revendications 1 à 11, **caractérisé en ce qu'**il comprend au moins les étapes suivantes :
- la transfection de lignées cellulaires cibles par au moins un plasmide contenant chacun une séquence hétérologue codant respectivement pour une chaîne A ou B selon l'une quelconque des revendications 1 à 11;
- l'expression et isolement des chaînes hétérologues A ou B ;
- la mise en milieu oxydant desdits polypeptides dans des proportions déterminées ;
- l'isolement des multimères.

16. Procédé selon la revendication 15 **caractérisé en ce que** les lignées transfectées ont été soit :
- co-transfectées.par deux plasmides porteurs de séquences d'ADN codant respectivement pour les polypeptides A et B, ou
- transfectées par un plasmide codant pour un premier polypeptide A puis sur-transfectées par le deuxième plasmide codant pour le deuxième polypeptide B, ou
- résultent de la fusion de cellules ayant respectivement été transfectées par un plasmide porteur d'une séquence d'ADN codant pour le polypeptide A et par un plasmide porteur d'une séquence d'ADN codant pour le polypeptide B.

17. Utilisation d'une protéine multimérique recombinante selon l'une quelconque des revendications 1 à 11 à la fabrication d'un médicament destiné à la prévention de l'allo-immunisation foeto-matemelle.

18. Utilisation d'une protéine multimérique recombinante selon l'une quelconque des revendications 1 à 11 à la fabrication d'un médicament destiné à la thérapeutique ou à la prophylaxie des infections virales, bactériennes ou parasitaires.

19. Utilisation d'une protéine multimérique recombinante selon l'une quelconque des revendications 1 à 11 à la fabrication d'un médicament destiné à la thérapeutique des maladies auto-immunes et notamment le lupus erythémateux disséminé.

20. Protéine multimérique recombinante selon l'une quelconque des revendications 1 à 11 pour utilisation dans un test de diagnostic nécessitant l'intervention d'au moins deux ligands différents.

21. Composition pharmaceutique **caractérisée en ce qu'**elle comprend en tant que principe actif une protéine multimérique selon l'une quelconque des revendications 1 à 11, ladite composition pharmaceutique permettant une immuno-thérapie ou une immuno-prévention de pathologies liées notamment aux infections virales, bactériennes, aux maladies auto-immunes ou allo-immune.

## Patentansprüche

1. Rekombinantes multimeres Protein, das eine Immunintervention bei einer humanen Dysimmunpathologie ermöglicht, **dadurch gekennzeichnet, dass** es mindestens umfasst:
a) ein Fusionsmonomer A polypeptidischer Natur, das aus einem C-terminalen Fragment der α-Kette von C4BP, gelegen zwischen den Aminosäuren 124 und 549, das Disulfidbrücken mit der C-terminalen Extremität der β-Kette von C4BP bildet, und aus einem Polypeptidfragment, das heterolog zur α-Kette ist, gebildet ist,
b) ein Fusionsmonomer B polypeptidischer Natur, das aus einem C-terminalen Fragment der β-Kette von C4BP, gelegen zwischen den Aminosäuren 120 und 235, das Disulfidbrücken mit der C-terminalen Extremität der α-Kette von C4BP bildet, und aus einem Polypeptidfragment, das heterolog zur β-Kette ist, gebildet ist,
wobei die Moleküle in a) und b) in ihrem C-terminalen Teil assoziiert sind, um das multimere Protein zu bilden.

2. Rekombinantes multimeres Protein gemäß Anspruch 1, **dadurch gekennzeichnet, dass** das C-terminale Fragment der α-Kette zwischen den Aminosäuren 493 und 549 liegt, und dass das C-terminale Fragment der β-Kette zwischen den Aminosäuren 176 und 235 liegt.

3. Rekombinantes multimeres Protein gemäß Anspruch 1 oder 2, **dadurch gekennzeichnet, dass** das Verhältnis der Anzahl Monomere α/β zwischen 7/1 und 5/3 liegt, und bevorzugt 7/1 beträgt.

4. Rekombinantes multimeres Protein gemäß einem der Ansprüche 1 bis 3, **dadurch gekennzeichnet, dass** die heterologen Fragmente in A und in B von spezifischen Liganden des Immunsystems stammen, besonders von Oberflächenproteinen von Lymphozyten vom Typ CD, von Antikörpern oder Antikörperfragmenten, von Antigenen oder Antigenfragmenten.

5. Rekombinantes multimeres Protein gemäß Anspruch 4, **dadurch gekennzeichnet, dass** es sich bei den Fragmenten, die von Lymphozytenproteinen stammen, um CD4, CD8, CD16, CD35 (oder CR1) handelt.

6. Rekombinantes multimeres Protein gemäß Anspruch 4, **dadurch gekennzeichnet, dass** die Antikörper oder Antikörperfragmente eine anti-Rh(D)-Spezifität aufweisen.

7. Rekombinantes multimeres Protein gemäß Anspruch 4, **dadurch gekennzeichnet, dass** es sich bei den Antigenen um Impfantigene handelt.

8. Rekombinantes multimeres Protein gemäß einem der Ansprüche 1 bis 3, **dadurch gekennzeichnet, dass** es sich bei dem heterologen Fragment in A um ein therapeutisches Enzym handelt.

9. Rekombinantes multimeres Protein gemäß einem der Ansprüche 1 bis 3, **dadurch gekennzeichnet, dass** die heterologen Polypeptidfragmente enthalten:
- in A das CD4 oder ein Derivat von CD4; und
- in B den scFv eines Antikörpers, insbesondere einen neutralisierenden Antikörper oder ein anti-Rh(D).

10. Rekombinantes multimeres Protein gemäß einem der Ansprüche 1 bis 3, **dadurch gekennzeichnet, dass** die heterologen Polypeptidfragmente enthalten:
- in A ein Antigen, besonders ein Impfantigen, oder ein therapeutisches Enzym oder CD35 (oder CR1) oder einen Antikörper, oder jedes Fragment davon, das die Ligandeneigenschaften des gesamten Moleküls besitzt,
- in B einen Antikörper oder ein Fragment davon, das sein Epitop bewahrt hat.

11. Rekombinantes multimeres Protein gemäß einem der Ansprüche 1 bis 3, **dadurch gekennzeichnet, dass** die heterologen Polypeptidfragmente enthalten:
- in A ein Impfimmunogen, und
- in B ein CD4 oder ein abgeleitetes Molekül, vorausgesetzt, dass es die Ligandeneigenschaft des gesamten Moleküls bewahrt.

12. Prokaryotische oder eukaryotische Zellen, **dadurch gekennzeichnet, dass** sie mit einem oder mehreren Plasmiden transfiziert wurden, die eine heterologe Nukleinsäuresequenz enthält bzw. enthalten, welche für mindestens ein polypeptidisches Fusionsmolekül A und ein polypeptidisches Fusionsmolekül B gemäß Anspruch 1 codiert.

13. Zellen gemäß Anspruch 12, **dadurch gekennzeichnet, dass** die Zellen entweder
- mit zwei verschiedenen Plasmiden cotransfiziert wurden, oder
- mit einem ersten Plasmid transfiziert wurden, das für ein erstes Polypeptid codiert, und anschließend mit dem zweiten Plasmid, das für das zweite Polypeptid codiert, über-transfiziert wurden, oder
- aus der Fusion der beiden Zellen stammen, von denen eine mit einem Plasmid transfiziert wurde, das für das erste Polypeptid codiert, und die andere mit einem Plasmid transfiziert wurde, das für das zweite Polypeptid codiert.

14. Zellen gemäß einem der Ansprüche 12 oder 13, **dadurch gekennzeichnet, dass** es sich bei dem ersten Plasmid um jenes handelt, das am 12. Juli 1995 bei der C.N.C.M. unter der Nummer 1-1610 hinterlegt wurde, und bei dem zweiten Plasmid um jenes handelt, das bei der C.N.C.M. am 12. Juli 1995 unter der Nummer 1-1611 hinterlegt wurde.

15. Verfahren zur Herstellung eines multimeren Proteins gemäß einem der Ansprüche 1 bis 11, **dadurch gekennzeichnet, dass** es mindestens die folgenden Schritte aufweist:
- Transfektion der Ziel-Zelllinien mit mindestens einem Plasmid, das jeweils eine heterologe Sequenz enthält, die für eine Kette A bzw. B gemäß einem der Ansprüche 1 bis 11 codiert;
- Expression und Isolierung der heterologen Ketten A oder B;
- Einführung der Polypeptide in ein oxidierendes Medium in vorbestimmten Verhältnissen;
- Isolierung der Multimere.

16. Verfahren gemäß Anspruch 15, **dadurch gekennzeichnet, dass** die transfizierten Linien entweder
- mit zwei Plasmiden cotransfiziert wurden, die Träger von DNA-Sequenzen sind, die für die Polypeptide A bzw. B codieren, oder
- mit einem Plasmid transfiziert wurden, das für ein erstes Polypeptid A codiert, und anschließend mit dem zweiten Plasmid, das für das zweite Polypeptid B codiert, über-transfiziert wurden, oder
- aus der Fusion der Zellen stammen, die mit einem Plasmid, das eine DNA-Sequenz trägt, die für das Polypeptid A codiert, bzw. mit einem Plasmid, das eine DNA-Sequenz trägt, die für das Polypeptid B codiert, transfiziert wurden.

17. Verwendung eines rekombinanten multimeren Proteins gemäß einem der Ansprüche 1 bis 11 bei der Herstellung eines Medikaments; das zur Prävention der fötalen-mütterlichen Alloimmunisierung bestimmt ist.

18. Verwendung eines rekombinanten multimeren Proteins gemäß einem der Ansprüche 1 bis 11 bei der Herstellung eines Medikaments, das zur Therapie oder Prophylaxe von viralen, bakteriellen oder parasitären Infektionen bestimmt ist.

19. Verwendung eines rekombinanten multimeren Proteins gemäß einem der Ansprüche 1 bis 11 bei der Herstellung eines Medikaments, das zur Therapie von Autoimmunerkrankungen und insbesondere Lupus erythematodes disseminatus bestimmt ist.

20. Rekombinantes multimeres Protein gemäß einem der Ansprüche 1 bis 11 zur Verwendung in einem diagnostischen Test, der den Einsatz von mindestens zwei verschiedenen Liganden erfordert.

21. Pharmazeutische Zubereitung, **dadurch gekennzeichnet, dass** sie als Wirkstoff ein multimeres Protein nach einem der Ansprüche 1 bis 11 umfasst, wobei die pharmazeutische Zubereitung eine Immuntherapie oder eine Immunprävention von Krankheiten ermöglicht, die insbesondere mit viralen, bakteriellen Infektionen oder Autoimmunerkrankungen oder Alloimmunerkrankungen in Verbindung stehen.

## Claims

1. Recombinant multimeric protein allowing an immunointervention in human dysimmunitary pathology, **characterized in that** it comprises at least:
a) a fusion monomer A, of polypeptide nature, which consists of a C-terminal fragment of the α chain of C4BP, contained between amino acids 124 and 549, forming disulfide bridges with the C-terminal end of the β chain of C4BP, and a polypeptide fragment which is heterologous in relation to said α chain,
b) a fusion monomer B, of polypeptide nature, which consists of a C-terminal fragment of the β chain of C4BP, contained between amino acids 120 and 235, forming disulfide bridges with the C-terminal end of the α chain of C4BP, and a polypeptide fragment which is heterologous in relation to the β chain,
the molecules in a) and b) being linked in their C-terminal portion in order to form said multimeric protein.

2. Recombinant multimeric protein according to claim 1, **characterized in that** the C-terminal fragment of the α chain is contained between amino acids 493 and 549, and **in that** the C-terminal fragment of the β chain is contained between amino acids 176 and 235.

3. Recombinant multimeric protein according to claim 1 or 2, **characterized in that** the ratio of the number of monomers α/β varies between 7/1 and 5/3 and is preferably 7/1.

4. Recombinant multimeric protein according to one of claims 1 to 3, **characterized in that** the heterologous fragments in A and in B are derived from specific ligands of the immune system, in particular derived from lymphocyte surface proteins of the CD type, from antibodies or antibody fragments, or from antigens or antigen fragments.

5. Recombinant multimeric protein according to claim 4, **characterized in that** the fragments derived from lymphocyte proteins are CD4, CD8, CD16 and CD35 (or CR1).

6. Recombinant multimeric protein according to claim 4, **characterized in that** the antibodies or antibody fragments have an anti-Rh (D) specificity.

7. Recombinant multimeric protein according to claim 4, **characterized in that** the antigens are vaccinating antigens.

8. Recombinant multimeric protein according to one of claims 1 to 3, **characterized in that** the heterologous fragment in A is a therapeutic enzyme.

9. Recombinant multimeric protein according to one of claims 1 to 3, **characterized in that** the polypeptide heterologous fragments contain:
- in A, CD4 or a derivative of CD4, and ;
- in B, the scFv of an antibody, in particular a neutralizing antibody or an anti-Rh (D) antibody.

10. Recombinant multimeric protein according to one of claims 1 to 3, **characterized in that** the polypeptide heterologous fragments contain:
- in A, an antigen, in particular a vaccinating antigen, or a therapeutic enzyme or a CD35 (or CR1) or an antibody, or any fragment thereof which possesses the ligand property of the whole molecule,
- in B, an antibody or a fragment thereof which has retained its epitope.

11. Recombinant multimeric protein according to one of claims 1 to 3, **characterized in that** the polypeptide heterologous fragments contain:
- in A, a vaccinating immunogen, and
- in B, a CD4 or a derived molecule which retains the ligand property of the whole molecule.

12. Prokaryotic or eukaryotic cells, **characterized in that** they have been transduced with one or more plasmids containing a heterologous nucleic acid sequence which encodes at least one polypeptide fusion molecule A and one polypeptide fusion molecule B according to claim 1.

13. Cells according to claim 12, **characterized in that** the cells have been either,
- cotransduced with two separate plasmids, or
- transduced with a first plasmid encoding a first polypeptide and then supertransduced with the second plasmid encoding the second polypeptide, or
- result from the fusion of two cells, one of which has been transduced with a plasmid encoding the first polypeptide while the other has been transduced with a plasmid encoding the second polypeptide.

14. Cells according to one of claims 12 or 13, **characterized in that** the first plasmid is that which was deposited in the C.N.C.M. under No. I-1610 on July 12, 1995, and the second plasmid is that which was deposited in the C.N.C.M. under No. I-1611 on July 12, 1995.

15. Process for preparing a multimeric protein as defined in any one of claims 1 to 11, **characterized in that** it comprises at least the following steps:
- transducing target cell lines with at least one plasmid, each of which contains a heterologous sequence which respectively encodes an A chain or a B chain according to any one of claims 1 to 11,
- expressing and isolating the heterologous A and B chains,
- placing said polypeptides, in selected proportions, in an oxidizing medium,
- isolating the multimers.

16. Process according to claim 15, **characterized in that** the transduced lines have been either:
- cotransduced with two plasmids carrying DNA sequences which respectively encode the A and B polypeptides, or
- transduced with one plasmid which encodes a first A polypeptide and supertransduced with the second plasmid which encodes the second B polypeptide, or
- result from the fusion of cells which have, respectively, been transduced with a plasmid carrying a DNA sequence which encodes the A polypeptide and with a plasmid carrying a DNA sequence which encodes the B polypeptide.

17. Use of a recombinant multimeric protein according to any one of claims 1 to 11 for producing a medicament which is intended for preventing foetomaternal alloimmunization.

18. Use of a recombinant multimeric protein according to any one of claims 1 to 11 for producing a medicament which is intended for the therapy or prophylaxis of viral, bacterial or parasitic infections.

19. Use of a recombinant multimeric protein according to any one of claims 1 to 11 for producing a medicament which is intended for the therapy of autoimmune diseases, in particular disseminated lupus erythematosus.

20. Recombinant multimeric protein according to any one of claims 1 to 11 for using in a diagnostic test which requires the intervention of at least two different ligands.

21. Pharmaceutical composition, **characterized in that** it comprises, as the active principle, a multimeric protein according to any one of claims 1 to 11, with said pharmaceutical composition enabling the immunotherapy or the immunoprevention of pathologies which are linked, in particular, to viral or bacterial infections or to autoimmune or alloimmune diseases.
